(19) Europäisches Patentamt
European Patent Office
Office européen des brevets

(11) Veröffentlichungsnummer: **0 193 056**
**B1**

(12) # EUROPÄISCHE PATENTSCHRIFT

(45) Veröffentlichungstag der Patentschrift: **07.06.89**

(51) Int. Cl.[4]: **A 61 K 31/46**, A 61 K 31/44 // (A61K31/46, 31:44),(A61K31/44, 31:135)

(21) Anmeldenummer: **86101942.0**

(22) Anmeldetag: **15.02.86**

(54) **Kombination von Flupirtin und anticholinergisch wirkenden Spasmolytika.**

(30) Priorität: **23.02.85 DE 3506508**
**22.03.85 DE 3510348**

(43) Veröffentlichungstag der Anmeldung: **03.09.86 Patentblatt 86/36**

(45) Bekanntmachung des Hinweises auf die Patenterteilung: **07.06.89 Patentblatt 89/23**

(84) Benannte Vertragsstaaten: **AT BE CH DE FR GB IT LI LU NL SE**

(56) Entgegenhaltungen:
**DE-A- 3 337 593**
**ARZNEIMITTELFORSCHUNG, Band 35 (I), Januar 1985, Seiten 30-43, Editio Cantor, Aulendorf/Württemberg, DE; V. JAKOVLEV et al.: "Untersuchungen zur pharmakologischen Wirkung von Flupirtin, einem strukturell neuartigen Analgetikum"**

(73) Patentinhaber: **ASTA Pharma Aktiengesellschaft**
**Weismüllerstrasse 45**
**D-6000 Frankfurt am Main 1 (DE)**

(72) Erfinder: **Tibes, Ulrich, Dr.**
**Am Sandberg 102**
**D-6000 Frankfurt/Main 70 (DE)**
Erfinder: **Weischer, Carl Heinrich, Dr.**
**Schmidtbonnstrasse 8**
**D-5300 Bonn 1 (DE)**
Erfinder: **Hettche, Helmut, Dr.**
**Buchrainweg 65**
**D-6050 Offenbach/Main (DE)**
Erfinder: **Breuel, Hans-Peter, Prof.**
**Am Jungstück 30**
**D-6500 Mainz 43 (DE)**
Erfinder: **Gunesch, Dietmar, Dr.**
**Matth.-Erzberger-Strasse 12**
**D-6050 Offenbach/Main (DE)**

EP 0 193 056 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Flupirtin ist ein Arzneimittelwirkstoff mit analgetischen Eigenschaften. Sein chemischer Name ist 2-Amino-3-carbethoxyamino-6-(4-fluor-benzylamino)-pyridin mit der folgenden Strukturformel :

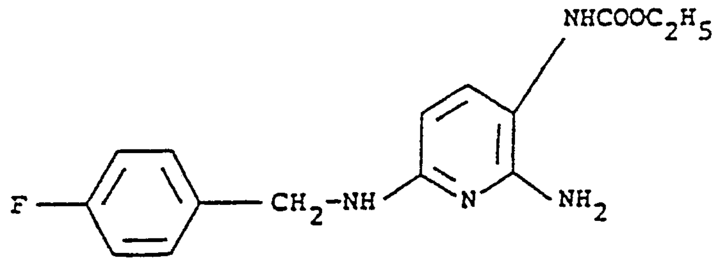

Das Flupirtin und dessen Salze mit physiologisch unbedenklichen Säuren besitzen eine ausgeprägte analgetische Hauptwirkung.

Es wurde nun gefunden, daß die Wirkung des Flupirtins und seiner Salze überraschenderweise durch Kombination mit Spasmolytika gesteigert wird, wobei gleichzeitig die Wirkung der Spasmolytika ebenfalls eine Steigerung erfährt. Die Wirkstoffe der erfindungsgemäßen Kombination potenzieren sich also gegenseitig in ihrer Wirkung.

Aufgabe der Erfindung ist die Bereitstellung von verbesserten Arzneimitteln mit analgetischer und spasmolytischer Wirkung.

Die in den Patentansprüchen angegebenen Gewichtsmengen beziehungsweise Gewichtsteile beziehen sich bei Flupirtin auf den reinen Wirkstoff (d. h. nicht auf Salze des reinen Wirkstoffs) bei den Spasmolytika auf die den Salzen jeweils zugrunde liegenden Kationen beziehungsweise Basen (letzteres z. B. beim Pramiverin).

Die Spasmolytika, die in Kombination mit dem Flupirtin verwendet werden, sind Spasmolytika mit ausgeprägter spasmolytischer Hauptwirkung und einer geringeren analgetischen Nebenwirkungskomponente. Es handelt sich hierbei um Spasmolytika, deren Wirkung darauf beruht, daß sie anticholinerg wirken.

Solche Spasmolytika sind beispielsweise Butylscopol- aminium-, Fenpiverinium-, Trospium-, Pramiverin- und Cicloniumsalze sowie deren Derivate.

Das Flupirtin wird vorzugsweise als Säureadditionssalz verwendet, wobei insbesondere die Salze mit Halogenwasserstoffsäuren (z. B. das Hydrochlorid) oder organischen Säuren (z. B. Maleat oder das Gluconat) in Frage kommen. Die Spasmolytika werden im allgemeinen in Form ihrer Halogensalze verwendet wie z. B. das Chlorid, Bromid, also z. B. :

Butylscopolaminiumbromid
Fenpiveriniumbromid
Trospiumchlorid
Pramiverin-Hydrochlorid
Cicloniumbromid

Das Flupirtin und die Spasmolytika können ganz allgemein in Form ihrer Salze mit organischen oder anorganischen Säuren, die zur Bildung von therapeutisch verwendbaren Salzen geeignet sind, für die Kombination verwendet werden. Als solche Säuren seien beispielsweise genannt : Halogenwasserstoffsäuren, Schwefelsäure, Phosphorsäuren, Salpetersäure, Perchlorsäure, organische Mono-, Di- oder Tricarbonsäuren der aliphatischen, alicyclischen, aromatischen oder heterocyclischen Reihe sowie Sulfonsäuren. Beispiele hierfür sind :

Ameisen-, Essig-, Propion-, Bernstein-, Glykol-, Milch-, Äpfel-, Wein-, Zitronen-, Ascorbin-, Malein-, Fumar-, Hydroxymalein- oder Brenztraubensäure ; Phenylessig-, Benzoe-, p-Aminosalicylsäure, Embonsäure, Methansulfon-, Ethansulfon-, Hydroxyethansulfon-, Ethylensulfonsäure ; Halogenbenzolsulfon-, Toluolsulfon-, Naphthalinsulfonsäure oder Sulfanilsäure oder Gluconsäure.

Die erfindungsgemäß zur Verwendung kommenden Spasmolytika enthalten ein basisches Stickstoffatom, welches in quartärer Form vorliegt. Wasserstoffatome, die sich an diesem basischen Stickstoffatom befinden, können auch durch eine oder zwei $C_1$-$C_6$-Alkylgruppen ersetzt sein. Ebenso können Alkylreste, die sich an diesem basischen Stickstoffatom befinden, durch einen oder zwei andere $C_1$-$C_6$-Alkylreste ersetzt sein. Derartige Verbindungen werden als Derivate oder Gruppe der erfindungsgemäß verwendbaren Spasmolytika bezeichnet. Die Alkylreste können gerade oder verzweigt sein. So kann beispielsweise im Falle des Butylscopolaminium-Kations der Methylrest und/oder der Butylrest an dem quartären basischen Stickstoffatom durch einen anderen $C_1$-$C_6$-Alkylrest (Methyl, Ethyl, Propyl, Isopropyl, Pentyl, Hexyl) ersetzt sein. Ebenso kann der Methylrest am basischen Stickstoffatom des Fenpiverinium-Kations durch einen anderen $C_1$-$C_6$-Alkylrest ersetzt sein. Das Analoge gilt z. B. für das Ciclonium-Kation. Beim Pramiverin kann z. B. das Wasserstoffatom an dem basischen Stickstoffatom durch einen $C_1$-$C_6$-Alkylrest ersetzt sein ; ebenfalls kann dann noch ein weiterer $C_1$-$C_6$-Alkylrest zur Bildung des entsprechenden quartären Salzes vorliegen.

Die Erfindung betrifft also Arzneimittel, enthaltend als Wirkstoff Flupirtin und mindestens ein anticholinergisch wirkendes Spasmolytikum, welches eine analgetische Nebenwirkungskomponente aufweist, wobei diese Wirkstoffe auch in Form ihrer Salze mit physiologisch unbedenklichen Säuren vorliegen können, wobei auf ein Gewichtsteil Flupirtin jeweils 0,05 bis 150, vorzugsweise 0,1 bis 100 Gewichtsteile Spasmolytikum kommen.

Die erfindungsgemäße Kombination zeigt beispielsweise überraschend in folgenden Versuchsmodellen eine potenzierte spasmolytische Wirkung, die gegenüber dem spasmolytisch wirksamen Anteil der Kombination überadditiv gesteigert ist :

Meerschweinchendünndarm in situ, Kohletransport-Modell am Meerschweinchen, Gallenblasen-Modell an der Maus, Rattenblase in situ[1)], Rattenuterus in situ[2)], Rattenuterus in vitro[3)].

Beispielsweise wird am Meerschweinchendünndarm in situ (siehe Tabelle 1) bei einer Dosis von 100 mg/kg intraduodenal Butylscopolaminiumbromid und 30 mg/kg intraduodenal Flupirtin die spasmolytische Wirksamkeit des Butylscopolaminiumbromids um den Faktor 2 gesteigert.

Demgegenüber zeigt das Butylscopolaminiumbromid (allein) bei intraduodenaler Verabreichung keine dosisabhängige spasmolytische Wirkung. Erst durch die Kombination mit Flupirtin wird eine erhebliche Wirkungssteigerung erreicht, wobei diese Wirkung nun dosisabhängig ist.

Flupirtin alleine intraduodenal appliziert, besitzt am Meerschweinchendünndarm keine spasmolytische Wirksamkeit. Um so überraschender ist es, daß beispielsweise nach der Kombinationsgabe von ansteigenden Mengen Flupirtin und einer konstanten Menge (100 mg/kg intraduodenal) Butylscopolaminiumbromid das Flupirtin ebenfalls eine deutliche spasmolytische Wirkung zeigt, aus der sich beispielsweise für das Flupirtin in der Kombination eine $ED_{50}$ von 19,5 mg/kg ergibt (Berechnung der $ED_{50}$ mittels linearer Regression).

Im Kohletransport-Modell am Meerschweinchen (siehe Tabelle 2) wird beispielsweise bei einer Dosis von 100 mg/kg per os Butylscopolaminiumbromid und 40 mg/kg per os Flupirtin-Hydrochlorid im Vergleich zu dem Versuch mit 40 mg/kg per os Flupirtin alleine verabreicht, der Kohletransport um den Faktor 2 gehemmt, bezogen auf die Wirksamkeit des Flupirtins allein. Die perorale $ED_{50}$ von Butylscopolaminiumbromid alleine verabreicht, beträgt beispielsweise an obigem Modell 132 mg/kg.

Die perorale $ED_{50}$ der spasmolytischen Komponente von Flupirtin in der Kombination beträgt beispielsweise 92 mg/kg. Hingegen konnte beispielsweise eine perorale $ED_{50}$ von Flupirtin, alleine verabreicht in einem Dosis-bereich von 20 bis 80 mg/kg, aufgrund zu schwacher und nicht dosisabhängiger Wirksamkeit nicht berechnet werden.

Es ist überraschend, daß die analgetische Nebenkomponente des Spasmolytikums wie auch die analgetische Hauptwirkung des Flupirtin gesteigert wird (Essigsäure-Writhing-Test an der Maus). Beispielsweise wird die analgetische Wirksamkeit von Flupirtin im Essigsäure-Writhing-Test (siehe Tabelle 3a) in der Kombination mit Butylscopolaminiumbromid überadditiv um den Faktor 31 gesteigert. Hierbei wird Butylscopolaminiumbromid bei 1 mg/kg per os konstant gehalten und Flupirtin in den Dosierungen 0,1 ; 0,5 ; 1 ; 2 und 4 mg/kg verabfolgt.

Beispielsweise wird die analgetische Wirksamkeit von Butylscopolaminiumbromid im Essigsäure-Writhing-Test in der Kombination mit Flupirtin um den Faktor 21 gesteigert. Hierbei wird Flupirtin bei 10 mg/kg per os konstant gehalten und Butylscopolaminium-bromid in den Dosierungen 0,01 ; 0,05 ; 0,1 ; 0,5 ; mg/kg verabfolgt. Das Flupirtin wurde hierbei als Maleat, Gluconat oder Hydrochlorid untersucht.

Das gleiche gilt zum Beispiel für die Kombination Flupirtin-Cicloniumbromid gemäß Tabelle 3b.

Beispielsweise liegen die bereits spasmolytisch wirksamen Dosen am Meerschweinchendünndarm in Maus für die Kombination bei 0,1 mg/kg per os Flupirtin und 1,0 mg/kg per os Butylscopolaminiumbromid.

Beispielsweise liegen die bereits spasmolytisch wirksamen Dosen am Meerschweinchendünndarn in situ für die Kombination bei 30 mg/kg intraduodenal Flupirtinund 50 mg/kg intraduodenal Butylscopolaminiumbromid oder 20 mg/kg intraduodenal Flupirtin und 100 mg/kg intraduodenal Butylscopolaminiumbromid.

Beispielsweise liegen die bereits spasmolytisch wirksamen Dosen am Kohletransport-Modell am Meerschweinchen für die Kombination bei 20 mg/kg per os Flupirtin und 100 mg/kg per os Butylscopolaminium-bromid.

Beispielsweise liegen die bereits spasmolytisch wirksamen Dosen am Gallenblasen-Modell an der Maus (siehe Tabelle 4) für die Kombination bei 3 mg/kg per os Flupirtin und 100 mg/kg per os Butylscopolaminiumbromid.

Beispielsweise kommen als Dosisbereiche der Kombination für die Wirkung am Writhing-Test (Maus) folgende in Betracht : 0,1-44 mg/kg Flupirtin und 0,01-2 mg/kg Butylscopolaminiumbromid.

[1)] Postius, S, I. Szelenyi, J. Pharmacol. Methods 9, 53-61 (1983).

[2)] in Anlehnung an die Methode gemäß [1)]

[3)] De Jalon, Bayo, De Jalon = Farmacoterap. act. 3, 313 (1945) ; Pharmacological Experiments on isolated Preparations 2nd ed. E + S Livingstone Edinburgh and London 1970, Seite 92-93.

In dem Essigsäure-Writhing-Test an der Maus kann beispielsweise das Gewichtsverhältnis von Flupirtin zu dem Spasmolytikum Butylscopolaminiumbromid das folgende sein :

1 Gewichtsteil Flupirtin auf 10 Gewichtsteile Spasmolytikum, vorzugsweise 1 Gewichtsteil Flupirtin auf 1 Gewichtsteil Spasmolytikum, insbesondere 1 Gewichtsteil Flupirtin auf 0,001 bis 0,05 Gewichtsteile Spasmolytikum.

Beispielsweise ist die synergistische Wirkung am gleichen Tiermodell insbesondere in folgendem Bereich der Gewichtsverhältnisse von Flupirtin und Spasmolytikum zu beobachten :

Flupirtin : Butylscopolaminiumbromid von 10 : 0,01 bis 0,1 : 1.

Die Gesamtdosis für die Kombination in den Tierversuchen liegt beispielsweise zwischen 1,1 mg/kg und 200 mg/kg vorzugsweise zwischen 1,1 und 130 mg/kg insbesondere zwischen 1,1 und 100 mg/kg per os.

Beispielsweise erhält man rechnerisch für die synergistische analgetische Wirkung von Flupirtin und Spasmolytikum am Writhing-Test an der Maus bei : 1,4 mg/kg per os Flupirtin und 1 mg/kg per os Butylscopolaminiumbromid oder 10 mg/kg per os Flupirtin und 0,07 mg/kg per os Butylscopolaminiumbromid eine 50 % Hemmung des Writhing-syndroms (= Schmerzhemmung). Beispielsweise erhält man rechnerisch für die spasmolytische Wirkung von Flupirtin und Spasmolytikum am Meerschweinchendünndarm in situ bei 30 mg/kg per os Flupirtin und 49 mg/kg per os Butylscopolaminiumbromid oder 100 mg/kg per os Butylscopolaminiumbromid und 19,5 mg/kg per os Flupirtin eine 50 % Hemmung des Acetylcholinspasmus.

Folgende Indikationen für die erfindungsgemäßen Kombinationen können in Betracht kommen : Akute und chronische spastische Schmerzzustände, Gallenkoliken, Spasmen im Bereich der Gallengänge, Gallenblasen- und Gallengangs-Dyskinesien, Nierenkoliken, Schmerzen im Bereich der ableitenden Harnwege, der Harnblase und der Urethra, spastische Schmerzen im Bereich des Magen-Darm-Traktes, Tenesmen, Dysmenorrhoe, Nabelkoliken, postoperative Schmerzzustände, spastische Schmerzen vor, während und nach diagnostischen Maßnahmen und therapeutischen Eingriffen, insbesondere im Bereich des Nierenbeckens und der ableitenden Harnwege, der Gallenblase und der Gallengänge sowie des Magen-Darm-Traktes einschließlich des Kolon und des Rektum.

Kontraindikationen : Engwinkelglaukom, Prostataadenom mit Restharnbildung, mechanische Stenosen im Bereich des Magen-Darmkanals, Tachyarrhythmie, Megacolon.

Die Tagesdosen der erfindungsgemäßen Kombination liegen beispielsweise bei 10 bis 900 mg (beispielsweise 10 bis 600 mg), vorzugsweise 50 bis 600 mg (beispielsweise 50 bis 400 mg), insbesondere 100 bis 400 mg (beispielsweise 100 bis 300 mg) Flupirtin und etwa 0,1 bis 150 mg, vorzugsweise 0,1 bis 100, insbesondere 0,5 bis 50 mg des Spasmolytikums (bezogen auf das basische Kation).

Die Tagesdosen können in Form einer einmaligen Verabreichung der gesamten Menge oder in Form von 1 bis 6 oder auch 1 bis 4 Teildosen pro Tag eingesetzt werden : Im allgemeinen ist eine Verabreichung 1 x täglich oder 3 bis 4 x täglich bevorzugt.

Für die Kombination von Flupirtin und Butylscopolaminiumbromid liegt die Tagesdosis im allgemeinen bei 100 bis 600 mg (beispielsweise 100 bis 500 mg) Flupirtin und etwa 10 bis 50 mg Butylscopolaminiumbromid 1 x täglich. Insbesondere liegt diese Dosis bei etwa 100 bis 400 mg Flupirtin und etwa 10 bis 30 mg Butylscopolaminiumbromid 1 x täglich. Bei mehreren Applikationen sind die genannten Dosen entsprechend zu teilen.

Im übrigen gelten hinsichtlich der spasmolytischen Komponenten die hierfür in der Literatur bekannten und vorgeschlagenen Tagesdosen (siehe beispielsweise Tabelle 5).

Vorzugsweise wird das Arzneimittel peroral verabreicht. Flupirtin und das jeweilige Spasmolytikum können in jeweils getrennten Formulierungen oder zusammen in einer galenischen Formulierung verwendet werden. Entsprechend einer bevorzugten Ausführung der Erfindung können die Arzneimittel in Form einer einzigen Dosis, das heißt in Form einer Mischung zur peroralen, parenteralen (intravenös, intramuskulär, subcutan), rektalen Verabfolgung formuliert werden, beispielsweise in Form von Tabletten, Kapseln, Pillen, Dragees, Suppositorien, Pellets, einer Lösung, Suspension oder Emulsion, wobei die Wirkstoffe mit entsprechenden Hilfs- und Trägerstoffen kombiniert werden. Bei der peroralen Form des Arzneimittels ist auch eine Retardformulierung möglich.

Die als Wirkstoffe dienenden Verbindungen, das heißt das Flupirtin und das jeweilige Spasmolytikum liegen in der Dosierungseinheit in einem Gewichtsverhältnis vor, daß auf ein Gewichtsteil Flupirtin 0,05 bis 150, vorzugsweise 0,1 bis 100, insbesondere 0,1 bis 10 Gewichtsteile des Spasmolytikums kommen. Hierbei beziehen sich die Gewichtsteile Spasmolytikum jeweils auf das zugrunde liegende basische Kation des Spasmolytikums beziehungsweise auf die freie Base des Spasmolytikums. Bei Verwendung der Salze der Spasmolytika gelten natürlich aufgrund des zusätzlichen Anions die jeweils auszurechnenden entsprechend höheren Gewichtsmengen.

Beispielsweise werden für die Kombinationen mit den Spasmolytika 1 bis 10 mg Butylscopolaminiumbromid und 1 bis 200 mg (beispielsweise 1 bis 150 mg) Flupirtin, vorzugsweise 2 bis 9 mg Butylscopolaminiumbromid und 10 bis 150 mg (beispielsweise 10 bis 100 mg) Flupirtin, insbesondere 3 bis 7 mg Butylscopolaminiumbromid und 15 bis 100 mg (beispielsweise 15 bis 60 mg) Flupirtin zum Arzneimittel formuliert.

Dies gilt nur für homogene Mischungen von Spasmolytikum und Flupirtin in den oben angegebenen Gewichtsverhältnissen. Diese Angaben sind nicht zwingend bei Kapseln und Zweischichtentabletten.

Die Dosierungseinheit der erfindungsgemäßen Kombination kann beispielsweise enthalten* :

a) bei peroralen Arzneiformen, welche ein Spasmolytikum aus der Gruppe der Butylscopolaminiumsalze enthalten : 50 bis 300, vorzugsweise 100 bis 200, insbesondere 100 bis 150 mg Flupirtin und 1 bis 40, (beispielsweise 2 bis 35 mg), vorzugsweise 2 bis 30 (beispielsweise 4 bis 20), insbesondere 5 bis 15 mg (beispielsweise 10 mg) Spasmolytikum. Diese Dosen können beispielsweise 1 bis 4, vorzugsweise 1 bis 3, insbesondere 1 bis 2 mal täglich verabreicht werden.

Bei peroralen Arzneiformen, welche ein Spasmolytikum aus der Gruppe der Trospiumsalze enthalten : 50 bis 300, vorzugsweise 100 bis 200, insbesondere 100 bis 150 mg Flupirtin und 1 bis 10, vorzugsweise 2 bis 8 mg, insbesondere 2 bis 4 mg Spasmolytikum. Diese Dosen können beispielsweise 1 bis 4, vorzugsweise 1 mal, insbesondere 1 bis 2 mal täglich verabreicht werden.

Bei peroralen Arzneiformen, welche ein Spasmolytikum aus der Gruppe der Pramiverinsalze enthalten : 50 bis 300, vorzugsweise 100 bis 200, insbesondere 100 bis 150 mg Flupirtin und 1 bis 10, vorzugsweise 1 bis 6, insbesondere 2 bis 4 mg Spasmolytikum.

Diese Dosen können beispielsweise 1 bis 4, vorzugsweise 1 bis 3, insbesondere 1 bis 2 mal täglich verabreicht werden.

Bei peroralen Arzneiformen, welche ein Spasmolytikum aus der Gruppe der Fenpiveriniumsalze enthalten : 50 bis 300, vorzugsweise 100 bis 200, insbesondere 100 bis 150 mg Flupirtin und 0,01 bis 1,0, vorzugsweise 0,05 bis 0,6, insbesondere 0,1 mg Spasmolytikum.

Diese Dosen können beispielsweise 1 bis 4, vorzugsweise 1 bis 3, insbesondere 1 bis 2 mal täglich verabreicht werden.

Bei peroralen Arzneiformen, welche ein Spasmolytikum aus der Gruppe der Cicloniumsalze enthalten : 50 bis 300, vorzugsweise 100 bis 200, insbesondere 100 bis 150 mg Flupirtin und 2 bis 80, vorzugsweise 4 bis 40, insbesondere 10 bis 20 mg Spasmolytikum.

Diese Dosen können beispielsweise 1 bis 4, vorzugsweise 1 bis 3, insbesondere 1 bis 2 mal täglich verabreicht werden.

b) Bei parenteralen Arzneiformen, welche ein Spasmolytikum aus der Gruppe der Butylscopolaminiumsalze enthalten : 50 bis 200, vorzugsweise 75 bis 150, insbesondere 100 mg Flupirtin und 0,01 bis 100, vorzugsweise 0,05 bis 50, insbesondere 0,1 bis 5 mg Spasmolytikum. Diese Dosen können beispielsweise 1 bis 6, vorzugsweise 1 bis 4, insbesondere 1 bis 2 mal täglich verabreicht werden.

Bei parenteralen Arzneiformen, welche ein Spasmolytikum aus der Gruppe der Trospiumsalze enthalten :

50 bis 200, vorzugsweise 75 bis 150, insbesondere 100 mg Flupirtin und 0,01 bis 1,0, vorzugsweise 0,1 bis 0,6, insbesondere 0,2 bis 0,4 mg Spasmolytikum. Diese Dosen können beispielsweise 1 bis 6, vorzugsweise 1 bis 4, insbesondere 1 bis 2 mal täglich verabreicht werden.

Bei parenteralen Arzneiformen, welche ein Spasmolytikum aus der Gruppe der Pramiverinsalze und Fenpiveriniumsalze enthalten : 50 bis 200, vorzugsweise 75 bis 150, insbesondere 100 mg Flupirtin und 0,5 bis 10 mg, vorzugsweise 1 bis 6, insbesondere 2 bis 4 mg Spasmolytikum. Diese Dosen können beispielsweise 1 bis 6, insbesondere 1 bis 4 mal oder auch 1 bis 2 mal täglich verabreicht werden.

Bei parenteralen Arzneiformen, welche ein Spasmolytikum aus der Gruppe der Cicloniumsalze enthalten :

50 bis 200, vorzugsweise 75 bis 150, insbesondere 100 mg Flupirtin, und vorzugsweise 2 bis 50, insbesondere 4 bis 25 mg Spasmolytikum. Diese Dosen können beispielsweise 1 bis 4, vorzugsweise 1 bis 3, insbesondere 1 bis 2 mal täglich verabreicht werden.

c) Bei rektalen Arzneiformen, welche ein Spasmolytikum aus der Gruppe der Butylscopolaminiumsalze enthalten : 75 bis 450, vorzugsweise 75 bis 300, insbesondere 100 bis 200 mg Flupirtin und 1 bis 40, vorzugsweise 2 bis 30, insbesondere 5 bis 15 (beispielsweise 10 mg) Spasmolytikum. Diese Dosen können beispielsweise 1 bis 4, insbesondere 1 bis 3 oder auch 1 bis 2 mal täglich verabreicht werden.

Bei rektalen Arzneiformen, welche ein Spasmolytikum aus der Gruppe der Pramiverinsalze enthalten : 75 bis 450, vorzugsweise 75 bis 300, insbesondere 100 bis 200 mg Flupirtin und 1 bis 20, vorzugsweise 2 bis 10, insbesondere 4 bis 8 mg Spasmolytikum. Diese Dosen können beispielsweise 1 bis 4, insbesondere 1 bis 3 mal oder auch 1 bis 2 mal täglich verabreicht werden.

Bei rektalen Arzneiformen, welche ein Spasmolytikum aus der Gruppe der Fenpiveriniumsalze enthalten : 75 bis 450, vorzugsweise 75 bis 300, insbesondere 100 bis 200 mg Flupirtin und 0,01 bis 1, vorzugsweise 0,01 bis 0,8 mg, insbesondere 0,1 bis 0,2 mg Spasmolytikum.

Diese Dosen können beispielsweise 1 bis 4, insbesondere 1 bis 3 mal täglich oder auch 1 bis 2 mal täglich verabreicht werden.

Bei rektalen Arzneiformen, welche ein Spasmolytikum aus der Gruppe der Cicloniumsalze enthalten : 75 bis 450, vorzugsweise 75 bis 300, insbesondere 100 bis 200 mg Flupirtin und 1 bis 100, vorzugsweise 2 bis 60 mg, insbesondere 5 bis 40 (beispielsweise 10 bis 20 mg) Spasmolytikum.

Diese Dosen können beispielsweise 1 bis 4, insbesondere 1 bis 3 mal oder auch 1 bis 2 mal täglich verabreicht werden.

Die akute Toxizität der erfindungsgemäßen Kombinationen an der Maus (ausgedrückt durch die

* Die angegebenen Gewichtsmengen für die Spasmolytika beziehen sich jeweils auf die Base beziehungsweise das basische Kation. Dasselbe gilt hinsichtlich des Flupirtins.

$LD_{50}$ mg/kg ; Methode Litchfield und Wilcoxon, J. Pharmacol. Exper. Ther. 95 : 99, 1949) liegt beispielsweise für die Kombination mit Flupirtin und Butylscopolaminiumbromid (1 : 1) bei oraler Applikation bei 618 mg/kg beziehungsweise oberhalb von 613 mg/kg Körpergewicht. Beispielsweise beträgt die $LD_{50}$ von Flupirtin alleine per os an der Maus : 552 mg/kg.

Beispielsweise enthält die bevorzugte Dosierungseinheit für die Kombination von Flupirtin und Butylscopolaminiumbromid 100 mg bis 600 mg (beispielsweise 100 bis 500 mg) Flupirtin und 10 bis 50 mg Butylscopolaminiumbromid. Insbesondere beträgt für die Kombination von Flupirtin und Butylscopolaminiumbromid diese Dosierungseinheit 100 bis 400 mg Flupirtin und 10 bis 30 mg Butylscopolaminiumbromid. Diese Dosierungseinheit kann zum Beispiel 1 bis 6, vorzugsweise 2 bis 4, insbesondere 2 bis 3 mal verabreicht werden.

Beispielsweise enthält die bevorzugte Dosierungseinheit für die Kombination von Flupirtin und Pramiverin-HCl 100 mg bis 600 mg (beispielsweise 100 bis 500 mg) Flupirtin und 1 bis 12 mg Pramiverin-HCl. Insbesondere beträgt für die Kombination von Flupirtin und Pramiverin-HCl diese Dosierungseinheit 100 bis 400 mg Flupirtin und 2 bis 4 mg Pramiverin-HCl.

Beispielsweise beträgt die bevorzugte Dosierungseinheit für die Kombination von Flupirtin und Trospiumchlorid 100 bis 600 mg (beispielsweise 100 bis 500 mg) Flupirtin und 0,2 bis 12 mg Trospiumchlorid. Insbesondere beträgt für die Kombination von Flupirtin und Trospiumchlorid diese Dosierungseinheit 100 bis 400 mg Flupirtin und 0,2 bis 5 mg Trospiumchlorid. Diese Dosierungseinheit kann zum Beispiel 1 bis 6, vorzugsweise 1 bis 4, insbesondere 2 bis 3 mal verabreicht werden.

Beispielsweise beträgt die bevorzugte Dosierungseinheit für die Kombination von Flupirtin und Fenpiveriniumbromid 100 bis 600 mg (beispielsweise 100 bis 500 mg) Flupirtin und 0,05 bis 0,8 mg Fenpiveriniumbromid. Insbesondere beträgt für die Kombination von Flupirtin und Fenpiveriniumbromid diese Dosierungseinheit 100 bis 400 mg Flupirtin und 0,1 bis 0,6 mg Fenpiveriniumbromid. Diese Dosierungseinheit kann zum Beispiel 1 bis 6, vorzugsweise 1 bis 4, insbesondere 2 bis 3 mal verabreicht werden.

Beispielsweise beträgt die bevorzugte Dosierungseinheit für die Kombination von Flupirtin und Cicloniumbromid 100 mg bis 600 mg (beispielsweise 100 bis 500 mg) Flupirtin und 10 bis 60 mg Cicloniumbromid. Insbesondere beträgt für die Kombination von Flupirtin und Cicloniumbromid diese Dosierungseinheit 100 bis 400 mg Flupirtin und 20 bis 40 mg Cicloniumbromid. Diese Dosierungseinheit kann zum Beispiel 1 bis 6, vorzugsweise 1 bis 4, insbesondere 2 bis 3 mal verabreicht werden.

Selbstverständlich können auch galenische Zubereitungen hergestellt werden, welche die oben angegebene Dosierungseinheit 2- bis beispielsweise 6 mal enthalten. So können beispielsweise Tabletten oder Kapseln der erfindungsgemäßen Kombination hergestellt werden, die 25-900 mg der Flupirtin-Komponente enthalten. (Bei der Verabreichung in Form von Granulaten, Pellets oder Pulvern (verpackt in Sachets) liegen beispielsweise 25 bis 4 000 mg Flupirtin vor.).

Die erfindungsgemäße Kombination ist zur Herstellung pharmazeutischer Zusammensetzungen und Zubereitungen geeignet. Die pharmazeutischen Zusammensetzungen beziehungsweise Arzneimittel enthalten als Wirkstoff Flupirtin in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können. Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind : Ullmanns Encyklopädie der technischen Chemie, Band 4 (1953), Seite 1 bis 39 ; Journal of Pharmaceutical Sciences, Band 52 (1963), Seite 918 u. ff., H. v. Czetsch-Lindenwald, Hilfsstoffe für Pharmazie und angrenzende Gebiete ; Pharm. Ind., Heft 2, 1961, Seite 72 u. ff. ; Dr. H. P. Fiedler, Lexikon der Hilfsstoffe für Pharmazie, Kosmetik und angrenzende Gebiete, 2. Auflage, Editio Cantor Aulendorf in Württemberg 1981.

Beispiele hierfür sind Gelatine, natürliche Zucker wie Rohrzucker oder Milchzucker, Lecithin, Pektin, Stärke (zum Beispiel Maisstärke) sowie Stärkederivate, Cyclodextrine und Cyclodextrinderivate, Polyvinylpyrrolidon, Gelatine, Gummi arabicum, Alginsäure, Tylose, Talkum, Lycopodium, Kieselsäure (zum Beispiel kolloidale), Cellulose, Cellulosederivate (zum Beispiel Celluloseether), bei denen die Cellulose-Hydroxygruppen teilweise mit niederen gesättigten aliphatischen Alkoholen und/oder niederen gesättigten aliphatischen Oxyalkoholen verethert sind (zum Beispiel Methylcellulose, Hydroxypropylmethylcellulose, Hydroxypropylmethylcellulosephthalat), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen und Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel: Polyacrylsäureester, Celluloseether und ähnliche.

Zur Herstellung von Lösungen und Suspensionen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxid, Fettalkohole, Triglyceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel: 1,3-Butandiol, Ethanol, 1,2-Propylenglykol, Polyglykole, vorzugsweise in Mischung mit Wasser, Ringer's Lösung, isotonische Kochsalzlösung oder auch Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxid-Kondensationsprodukte von Fettalkoholen, Alkylphenolen oder Fettsäuren oder auch 1-Methyl-3-(2-hydroxyethyl)-imidazolin-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt. Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxyd erhalten werden (zum Beispiel 40 Mol Ethylenoxide pro Mol Glycerid).

Beispiele für Oleotriglyceride sind Olivenöl, Erdnussöl, Rizinusöl, Sesamöl, Baumwollsaatöl, Maisöl. Siehe auch Dr. H. P. Fiedler « Lexikon der Hilfsstoffe für Pharmazie, Kosmetik und angrenzende Gebiete » 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süssmitteln, Farbstoffen, Antioxydantien und Komplexbildnern (zum Beispiel Ethylendiaminotetraessigsäure) und dergleichen möglich.

Gegebenenfalls ist zur Stabilisierung der Wirkstoffmoleküle mit physiologisch verträglichen Säuren oder Puffern auf einen pH-Bereich von ca. 2 bis 8 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Aceton- Natriumdisulfit, Gallussäure, Gallussäure-alkylester, Butylhydroxyanisol, Nordihydroguajaretsäure, Tocopherole sowie Tocopherole + Synergisten (Stoffe, die Schwermetalle durch Komplexbildung binden, beispielweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Nieder-alkylester), Benzoesäure, Natriumbenzoat, Trichlorisobutylalkohol, Phenol, Kresol, Benzethonium und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80 °C, vorzugsweise 20 bis 50 °C, insbesondere bei Raumtemperaturen gearbeitet wird. Im übrigen wird auf folgende Standardwerke verwiesen: Sucker, Fuchs, Speiser, Pharmazeutische Technologie, Thieme-Verlag Stuttgart, 1978; Voigt, Lehrbuch der pharmazeutischen Technologie, 3. Auflage, Verlag Chemie, Weinheim und New York, 1979; List, Arzneiformenlehre, Wissenschaftliche Verlagsgesellschaft mbH, Stuttgart, 1976.

Beschreibung der angeführten pharmakologischen Testmodelle

Kohletransport-Modell (Einfluß auf die Magen-Darm-Passage, Meerschweinchen)

In Anlehnung an KOMLOS und PETÖCZ[1)] (1970) Arzneim. Forsch. 20, 1338-1357 erhielten Meerschweinchen 30 Minuten nach der Substanzgabe eine 10 %ige Kohlesuspension in entmineralisiertem Wasser in einem Volumen von 1 ml/Tier mit der Magensonde verabreicht. 0,5 Stunden später wurden die Tiere getötet und, vom Magen ausgehend, einmal die Gesamtlänge des Darms, zum anderen die von der Kohle geschwärzte Strecke gemessen. Die von der Kohle durchwanderte Strecke wurde in Prozent der Gesamtlänge umgerechnet, um die verschiedene Darmlänge der einzelnen Tiere bei der Beurteilung der Substanzwirkung auszugleichen. Die Wirkung auf die Magen-Darm-Passage ergab sich aus dem

1) und Modifikation

Vergleich zwischen den Kontroll- und den mit den Substanzen behandelten Tieren (Mittelwerte). Ermittlung der $ED_{50}$ (Dosis in mg/kg bei der 50 % Hemmung des Kohletransports vorliegt) mittels linearer Regression.

Hemmung des Kohletransports bedeutet spasmolytischer Effekt. Je größer diese Hemmung desto größer ist auch der spasmolytische Effekt.

Essigsäure-Test (Writhing-Test) an der Maus :

Im Essigsäure-Test nach Koster et. al. (Fed. Proc., Band 18, 1959, Seite 412) wird der Schmerzreiz durch eine intraperitoneale Injektion verdünnter Essigsäure (1 %ig) ausgelöst. Die Schmerzreaktion äußert sich als ein charakteristisches Strecken der Tiere (« writhing syndrom »), das in unregelmäßigen Zeitabständen längere Zeit nach der Essigsäure-Injektion anhält. Die dosisabhängige Hemmung der Häufigkeit der Streckbewegungen 30 Minuten nach Essigsäureinjektion gegenüber einer unbehandelten Kontrollgruppe wird als analgetische Wirkung in Prozent ausgedrückt. Die Auswertung erfolgt durch Bestimmung der $ED_{50}$ (Methode der linearen Regression). Die $ED_{50}$ ist die Dosis in mg/kg, bei der rechnerisch eine 50 %ige Hemmung des « Writhingsyndroms » vorliegt.

Der Essigsäure-Test zeichnet sich dadurch aus, daß nicht nur die Wirkung von starken, zentral wirksamen Analgetika, sondern auch von überwiegend peripher wirksamen Analgetika-Antipyretika und antiphlogistisch wirksamen Pharmaka wie Phenylbutazon, Indometacin und andere nachweisbar ist. Damit weist die Wirkung in dieser Versuchsanordnung auf eine periphere Komponente der Analgesie hin.

Spasmolyse am Meerschweinchendünndarm

Mit Urethan narkotisierten Meerschweinchen (1,8 g/kg subkutan) wird nach Tracheotomie (Einbinden einer Trachealkanüle zur Atmungserleichterung) ein Ballonkatheter in das Ileum eingeführt und mit Luft gefüllt.

Der Darm führt rhytmische Kontraktionen aus, die sogenannte Peristaltik (füllungsabhängig), wodurch ein Druck auf den Gummiballon ausgeübt wird. Die ausgeübten Drücke und deren Veränderungen werden über Druckaufnehmer an den der Ballonkatheter angeschlossen ist und Trägerfrequenzbrücke (TF der Fa. Hellige) auf einem Linearschreiber aufgezeichnet. Es kann zum einen die Spontanperistaltik, zum anderen die durch Superfusionsgabe von Spasmodika (Acetylcholinjodid, Bariumchlorid oder Carbachol) ausgelösten Spasmen gemessen werden. Durch vorhergehende intravenöse oder intraduodenale Gabe von Spasmolytika wird die Höhe der ausgelösten Spasmen gehemmt und kann in Prozent Spasmolyse gegenüber einem Ausgangswert ermittelt werden. Als spasmolytischer Effekt beziehungsweise Spasmolyse wird angegeben, um wieviel Prozent der durch das Spasmogen (z. B. Acetylcholinjodid) ausgelöste Spasmus gehemmt ist gegenüber dem Wert, der von dem Spasmogen herrührt (am gleichen Tier).

Ermittlung der $ED_{50}$ (Dosis in mg/kg, bei der 50 % Spasmolyse vorliegt) mittels linearer Regression.

Gallenblasen-Modell an der Maus

Der Versuch wird in Anlehnung an die Methode von Valsecchi und Toson J. Pharmacol. Methods 7, 193 ff. (1982), durchgeführt.

Das Prinzip dieser Methode ist die gravimetrische Messung der Gallenblase der Maus.

Durch Verabreichung von Eigelb wird die Gallenblase zur Abgabe von Gallenflüssigkeit angeregt. Diese Abgabe erfolgt durch natürliche Spasmen der Gallenblase. Durch vorherige Verabreichung einer spasmolytisch wirkenden Testsubstanz werden diese Spasmen der Gallenblase gehemmt und damit ebenfalls die Abgabe der Gallenflüssigkeit.

Je schwerer also die Gallenblase nach der Eigelb-Verabreichung ist, desto weniger Gallenflüssigkeit hat sie abgegeben. Das Gewicht der Gallenblase bei diesem Versuch ist daher ein Maß für den spasmolytischen Effekt einer Substanz.

Die zu prüfenden Substanzen werden in 1 %iger Methocelsuspension peroral an die Tiere verabreicht. Pro Dosis wurden 10 Tiere eingesetzt. Die Kontrolltiere erhalten Methocel in der entsprechenden Menge.

Applikationsvolumen : 30 ml/kg. Aus Gründen der Auswertung müssen 2 Kontrollgruppen vorhanden sein.

15 Minuten nach Substanzgabe erhalten Tiere der Kontrollgruppe Nr. 1 1 ml NaCl 0,9 %/Tier peroral, die andere Kontrollgruppe Nr. 2 sowie die Substanzgruppen erhalten 1 ml Eigelbsuspension 30 %/Tier peroral.

Die Eigelbsuspension besteht aus 3 Gewichtsteilen Eigelb in 7 Gewichtsteilen NaCl 0,9 % ; aus diesem Grund erhält auch Kontrollgruppe Nr. 1 1 ml NaCl 0,9 %.

30 Minuten nach Substanzgabe werden die Tiere mittels Ether getötet. Die Gallenblase wird präpariert und gewogen.

Statistik

Die Auswertung erfolgte nach der Formel :

$$(A - B)/(C - B) \times 100 = \%\ \text{Wirkung}$$

wobei A den arithmetischen Mittelwert der jeweiligen Substanzgruppe bezeichnet, B den der Eigelbkontrolle und C den der Methocelkontrolle (Kontrollgruppe Nr. 1) darstellt.

Literatur

B. Valsecchi und G. Toson :
Journal of Pharmacological Methods 7
193-195 (1982)

Tabelle 1

Wirkung am Meerschweinchendünndarm in situ

(Spasmogen : Acetylcholinjodid 1 × $10^{-4}$ g/ml)

| Wirkstoff, Kombination | Dosis (mg/kg) intraduodenal | Anzahl der Tiere | % spasmolytischer Effekt (negativer Wert) | $ED_{50}$ in mg/kg 30 Minuten nach Verabreichung |
|---|---|---|---|---|
| Flupirtin (Maleat) allein | 10.0 | 6 | kein spasmolytischer Effekt. | |
| | 30.0 | 6 | | |
| | 50.0 | 4 | | |
| Butylscopolaminiumbromid allein | 50.0 | 6 | - 13.1% | diese Hemmung ist nicht dosisabhängig und daher eine $ED_{50}$ nicht bestimmbar. |
| | 100.0 | 6 | - 37.9% | |
| | 200.0 | 6 | - 35.0% | |
| | Butylscopolaminiumbromid | | | von Butylscopolaminiumbromid |
| Flupirtin (Maleat, 30.0 mg/kg)+ Butylscopolaminiumbromid | 10.0 | 6 | - 8.8% | $ED_{50}$ |
| | 50.0 | 6 | - 45.1% | 48.6 mg/kg |
| | 100.0 | 6 | - 73.5% | |
| 100.0 mg/kg Butylscopolaminiumbromid + Flupirtin (Maleat) | Flupirtin(Maleat) | | | von Flupirtin (Maleat) |
| | 10.0 | 6 | - 12.3% | $ED_{50}$ |
| | 20.0 | 6 | - 52.5% | 19.48 mg/kg |
| | 30.0 | 6 | - 73.5% | |

Methode : Berechnung der $ED_{50}$ mittels linearer Regression

Tabelle 2

Wirkung im Kohletransport-Modell beim Meerschweinchen
In jeder Dosisgruppe wurden 6 Tiere verwendet.

| Wirkstoff, Kombination | Dosis (mg/kg) peroral | spasmolytischer Effekt: Hemmung in % (negative Werte:) | spasmolytischer Effekt: $ED_{50}$ (*) in mg/kg |
|---|---|---|---|
| | Flupirtin (HCl) | | |
| Flupirtin (HCl) allein | 20.0 | - 11.73% | nicht dosisabhängiger, nur sehr schwacher Effekt |
| | 40.0 | - 21.55% | |
| | 80.0 | - 18.70% | |
| | Butylscopolaminiumbromid | | von Butylscopolaminiumbromid |
| Butylscopolaminiumbromid allein | 30.0 | - 8.16% | 131.6 |
| | 100.0 | - 58.39% | |
| | 300.0 | - 61.62% | |
| Butylscopolaminiumbromid (100 mg/kg) + Flupirtin (HCl) | Flupirtin (HCl) | | von Flupirtin (HCl) |
| | 1.0 | - 1.3% | 91.8 |
| | 10.0 | + 5.1% | |
| | 20.0 | - 35.2% | |
| | 40.0 | - 45.0% | |
| | 80.0 | - 50.3% | |
| | 100.0 | - 54.3% | |

(*) Berechnung der $ED_{50}$ mittels linearer Regression

Tabelle 3a

Analgetische Wirkung im Writhing-Test (NMRI-Maus)
(Koster et. al. : Fed. Proc. 18, 412 (1959))

| Wirkstoff, Kombination | Dosis (mg/kg) per os | Wirkung in % Mittel von je 10 Mäusen | $ED_{50}$ nach 30 Minuten in mg/kg |
|---|---|---|---|
| Flupirtin (Gluconat) allein | 0.56 | 19.2% | Flupirtin (Gluconat) |
| | 1.12 | 28.0% | |
| | 2.24 | 37.6% | |
| | 4.47 | 46.4% | 43.9 |
| | 8.93 | 38.4% | |
| | 17.85 | 22.4% | |
| | 35.7 | 61.6% | |
| Flupirtin (Maleat) allein | 20.0 | 12.0% | Flupirtin (Maleat) |
| | 40.0 | 54.0% | 44.7 |
| | 80.0 | 70.0% | |
| Butylscopolaminiumbromid allein | | | Butylscopolaminiumbromid |
| | 0.5 | 22.4% | |
| | 1.0 | 48.4% | 1.5 |
| | 2.0 | 64.6% | |
| | 4.0 | 59.0% | |
| 10 mg/kg Flupirtin (HCl) | Butylscopolaminiumbromid | | von Butylscopolaminiumbromid |
| + Butylscopolaminiumbromid | 0.01 | 25.2% | |
| | 0.05 | 48.3% | |
| | 0.1 | 60.1% | 0.07 |
| | 0.5 | 68.5% | |
| 1.0 mg/kg | Flupirtin (HCl) | | von Flupirtin (HCl) |
| Butylscopolaminiumbromid | 0.1 | 30.8% | |
| + Flupirtin (HCl) | 0.5 | 36.8% | |
| | 1.0 | 53.4% | 1.4 |
| | 2.0 | 52.6% | |
| | 4.0 | 56.4% | |

Methode 1 : Berechnung der $ED_{50}$ mittels linearer Regression

Tabelle 3b

Analgetische Wirkung im Writhing-Test (NMRI-Maus)
Koster et al., Fed. Proc. 18, 412 (1959)

| Wirkstoff, Kombination | Dosis (mg/kg) per os | Wirkung in % Mittel von je 10 Mäusen | $ED_{50}$ nach 30 Minuten in mg/kg |
|---|---|---|---|
| Cicloniumbromid allein | 0.01 | 18.5 | Cicloniumbromid allein |
| | 0.1 | 46,3 | |
| | 0.5 | 53,1 | 0.26 |
| Cicloniumbromid + 2.24 mg/kg Flupirtin (HCl) | Cyclonium-bromid | | Cicloniumbromid |
| | 0.001 | 33.1 | |
| | 0.01 | 53.1 | 0.01 |
| | 0.05 | 58.1 | |

Bestimmung der $ED_{50}$: mittels linearer Regression

Tabelle 4

Wirkung am Gallenblasen-Modell an der NMRI-MAUS
(Valsecchi and Toson, J. Pharmacol. Methods 7, 193-195, 1982)
In jeder Dosisgruppe wurden 10 Tiere verwendet

| Wirkstoff, Kombination | Dosis (mg/kg) peroral | spasmolytischer Effekt in % | $ED_{50}$ (*) in mg/kg |
|---|---|---|---|
| | Flupirtin (HCl) | | Flupirtin (HCl) |
| Flupirtin (HCl) | 1.0 | 30.06% | |
| allein | 3.0 | 64.36% | 2.4 |
| | 10.0 | 69.25% | |
| Butylscopolaminium- | | | von Butylscopolaminiumbromid |
| bromid | 3.0 | 4.6% | |
| allein | 10.0 | 19.1% | |
| | 30.0 | 20.7% | 116.6 |
| | 100.0 | 35.9% | |
| | 300.0 | 76.6% | |
| 3 mg/kg Flupirtin (HCl) | Butylscopolaminiumbromid | | von Butylscopolaminiumbromid |
| + Butylscopolami- | | | |
| niumbromid | 1.0 | 2.70% | |
| | 10.0 | 38.30% | |
| | 30.0 | 24.10% | 100.2 |
| | 100.0 | 54.80% | |
| Butylscopolaminium- | Flupirtin (HCl) | | von Flupirtin (HCl) |
| bromid (100 mg/kg) | | | |
| + Flupirtin (HCl) | 1.0 | 23.7% | |
| | 3.0 | 31.9% | 3.7 |
| | 10.0 | 65.5% | |
| | 30.0 | 125.6% | |

(*) Berechnung der $ED_{50}$ mittels linearer Regression

Tabelle 5

Dosen für die spasmolytische Komponente

| Substanz | Tagesdosen | Einzeldosen | tägliche Applikations-häufigkeit |
| --- | --- | --- | --- |
| Butylscopolami-niumbromid Dragees | 30 - 50 mg | 10 mg | 3 - 5 |
| Butylscopolami-niumbromid Suppositorien | 10 - 20 mg | 10 mg | 1 - 2 |
| Butylscopolami-niumbromid Ampulle | 20 mg | 20 mg | 1 |
| Pramiverin Ampulle | 2,25 - 4,5 mg | 2,25 mg | 1 - 2 |
| Pramiverin Tropfen | 25 Tropfen 3 x täglich | 1 ml = 2 mg | 3 - 4 |
| Pramiverin Suppositorien | 6 - 12 mg | 6 mg | 1 - 2 |
| Trospiumchlorid Ampulle | 0,2 - 0,4 mg | 2 ml = 0,2 mg | 1 - 2 |
| Trospiumchlorid Suppositorien | 1 - 5 mg | 1 mg | 1 - 5 |
| Trospiumchlorid Tabletten | 2 - 12 mg | 2 mg | 1 - 3 |
| Fenpiverinium-bromid Ampulle | 2 - 5 ml<br>0,05 - 0,1 mg | 5 ml = 0,1 mg | 1 - 2 |
| Fenpiverinium-bromid Tabletten | 0,1 - 0,8 | 0,1 mg | 1 - 4 |
| Fenpiverinium-bromid Suppositorien | 0,2 - 0,3 mg | 0,1 mg | 2 - 3 |
| Cicloniumbromid Ampulle | 12,5 - 25 mg | 1 ml = 5 mg | 1 |
| Cicloniumbromid Tabletten | 20 - 60 mg | 10 mg | 2 - 3 |
| Cicloniumbromid Suppositorien | 40 - 60 mmg | 20 mg | 2 - 3 |

Beispiel 1

Kapseln mit 40 mg Flupirtinmaleat und 5 mg Butylscopolaminiumbromid :

80 g Flupirtinmaleat werden mit 10 g Butylscopolaminiumbromid und 160 g Cellulose gemischt und anschließend mit einer Lösung von 3 g Kollidon® VA 64* in 115 ml Wasser in üblicher Weise granuliert. Das getrocknete Granulat wird durch ein Sieb der Maschenweite 0,8 mm gegeben und anschließend mit 4 g Magnesiumstearat, 1 g Hochdispersem Siliciumdioxid und 42 g modifizierter Stärke** gemischt.

Die Mischung wird zu jeweils 150 mg in Hartgelatinekapseln der Größe 3 gefüllt.

Eine Kapsel enthält 40 mg Flupirtinmaleat und 5 mg Butylscopolaminiumbromid.

Analog können Kapseln hergestellt werden, die zum Beispiel 100 mg Flupirtinmaleat und 10 mg N-Butylscopolaminiumbromid enthalten.

Beispiel 2

Suppositorien mit 40 mg Flupirtinmaleat und 5 mg Butylscopolaminiumbromid :

20 g Flupirtinmaleat und 2,5 g Butylscopolaminiumbromid werden in 997,5 g geschmolzenem Hartfett*** suspendiert. Nach Homogenisierung wird die Suspension in üblicher Weise in Hohlzellen von 2,3 ml ausgegossen und abgekühlt.

Ein Suppositorium vom Gewicht 2,04 g enthält 40 mg Flupirtinmaleat und 5 mg Butylscopolaminiumbromid.

Analog können zum Beispiel Suppositorien hergestellt werden, die zum Beispiel 150 mg Flupirtinmaleat und 10 mg N-Butylscopolaminiumbromid enthalten.

Beispiel 3

Kapseln mit 40 mg Flupirtinmaleat und 0,1 mg Fenpiveriniumbromid :

80 g Flupirtinmaleat werden mit 169,8 g Cellulose gemischt und mit einer Lösung aus 0,2 g Fenpiveriniumbromid und 3 g Kollidon® VA 64 in 120 ml Wasser in üblicher Weise granuliert. Das getrocknete Granulat wird durch ein Sieb der Maschenweite 0,8 mm gegeben und anschließend mit 4 g Magnesiumstearat, 1 g Hochdispersem Siliciumdioxid und 42 g modifizierter Stärke gemischt. Die Mischung wird zu jeweils 150 mg in Hartgelatinekapseln der Größe 3 gefüllt.

Eine Kapsel enthält 40 mg Flupirtinmaleat und 0,1 mg Fenpiveriniumbromid.

Beispiel 4

Kapseln mit 40 mg Flupirtinmaleat und 2 mg Pramiverin. HCl :

80 g Flupirtinmaleat werden mit 166 g Cellulose gemischt und mit einer Lösung aus 4 g Pramiverin. HCl und 3 g Kollidon® VA 64 in 115 ml Wasser in üblicher Weise granuliert.

Das getrocknete Granulat wird durch ein Sieb der Maschenweite 0,8 mm gegeben und anschließend mit 4 g Magnesiumstearat, 1 g Hochdispersem Siliciumdioxid und 42 g modifizierter Stärke gemischt. Die Mischung wird zu jeweils 150 mg in Hartgelatinekapseln der Größe 3 gefüllt.

Eine Kapsel enthält 40 mg Flupirtinmaleat und 2 mg Pramiverin. HCl.

Beispiel 5

Suppositorien mit 40 mg Flupirtinmaleat und 6 mg Pramiverin. HCl :

20 g Flupirtinmaleat und 3 g Pramiverin. HCl werden in 997 g geschmolzenem Hartfett suspendiert. Nach Homogenisierung wird die Suspension in üblicher Weise in Hohlzellen von 2,3 ml ausgegossen und abgekühlt.

Ein Suppositorium vom Gewicht 2.04 g enthält 40 mg Flupirtinmaleat und 6 mg Pramiverin. HCl.

Beispiel 6

Tabletten mit 75 mg Flupirtinmaleat und 10 mg Cicloniumbromid :

300 g Flupirtinmaleat werden mit 40 g Cicloniumbromid gemischt und die Mischung mit einem Schleim aus 20 g Maisstärke in 370 g Wasser in üblicher Weise granuliert. Nach dem Trocknen wird das Granulat durch ein Sieb der Maschenweite 0,8 mm gegeben und anschließend mit 300 g Mikrokristalliner Cellulose, 52 g modifizierter Stärke (wie in Beispiel 1), 7 g Magnesiumstearat sowie 1 g Hochdispersem

* Kollidon VA 64 ist ein Vinylpyrrolidon-Vinylacetat-Copolymerisat 60 : 40.

** Bei der modifizierten Stärke handelt es sich um eine freifließende und teilweise kaltwasserlösliche Maisstärke ; diese Modifikation erfolgt durch rein physikalische Maßnahmen.

*** Hartfett ist ein Gemisch von Mono-, Di- und Triglyceriden der gesättigten Fettsäuren von $C_{10}H_{20}O_2$ bis $C_{18}H_{36}O_2$.

Siliciumdioxid (Aerosil® 200) gemischt. Die Mischung wird zu Tabletten vom Gewicht 180 mg, einem Durchmesser von 8 mm und einem Wölbungsradius von 8 mm verpreßt. Anschließend können die Tabletten gegebenenfalls in der üblichen Weise mit einem Filmüberzug versehen werden.

Eine Tablette enthält 75 mg Flupirtinmaleat und 10 mg Cicloniumbromid.

Beispiel 7

Suppositorien mit 75 mg Flupirtinmaleat und 10 mg Cicloniumbromid :

37,5 g Flupirtinmaleat und 5 g Cicloniumbromid werden in 982,5 g geschmolzenem Hartfett suspendiert. Nach Homogenisierung wird die Suspension in üblicher Weise in Hohlzellen von 2,3 ml ausgegossen und abgekühlt.

Ein Suppositorium vom Gewicht 2,05 g enthält 75 mg Flupirtinmaleat und 10 mg Cicloniumbromid.

**Patentansprüche** (für die Vertragsstaaten : BE, CH, DE, FR, GB, IT, LI, LU, NL, SE)

1. Arzneimittel, enthaltend als Wirkstoff Flupirtin und mindestens ein anticholinergisch wirkendes Spasmolytikum, welches eine analgetische Nebenwirkungskomponente aufweist, wobei diese Wirkstoffe auch in Form ihrer Salze mit physiologisch unbedenklichen Säuren vorliegen können und wobei auf ein Gewichtsteil Flupirtin jeweils 0,05 bis 150, vorzugsweise 0,1 bis 100 Gewichtsteile Spasmolytikum kommen.

2. Mittel nach Anspruch 1, dadurch gekennzeichnet, daß das Spasmolytikum ein Butylscopolaminiumsalz, Fenpiveriniumsalz, Trospiumsalz, Pramiverinsalz oder Cicloniumsalz ist.

3. Mittel nach einem oder mehreren der vorangegangenen Ansprüche, dadurch gekennzeichnet, daß die Kombination 10 bis 900 mg Flupirtin und 0,3 bis 100 mg des Spasmolytikums enthält.

4. Mittel nach einem oder mehreren der vorangegangenen Ansprüche, dadurch gekennzeichnet, daß die Kombination 10 bis 600 mg Flupirtin und 0,3 bis 100 mg des Spasmolytikums enthält.

5. Mittel nach einem oder mehreren der vorangegangenen Ansprüche, dadurch gekennzeichnet, daß die Kombination 30 bis 600 mg Flupirtin und 3 bis 60 mg Butylscopolamin (als Kation) enthält.

6. Mittel nach einem oder mehreren der vorangegangenen Ansprüche, dadurch gekennzeichnet, daß die Kombination 30 bis 400 mg Flupirtin und 3 bis 60 mg Butylscopolamin (als Kation) enthält.

7. Mittel nach einem oder mehreren der vorangegangenen Ansprüche, dadurch gekennzeichnet, daß die Kombination 10 bis 900 mg Flupirtin und 5 bis 60 mg Ciclonium-Base (als Kation) enthält.

**Patentansprüche** (für den Vertragsstaat AT)

1. Verfahren zur Herstellung von Arzneimitteln, dadurch gekennzeichnet, daß man Flupirtin und mindestens ein anticholinergisch wirkendes Spasmolytikum oder deren Salze mit physiologisch unbedenklichen Säuren, gegebenenfalls mit weiteren üblichen Träger- und/oder Verdünnungs- beziehungsweise Hilfsstoffen zu pharmazeutischen Zubereitungen formuliert, wobei man 0,05 bis 150 Gewichtsteile Spasmolytikum auf einen Gewichtsteil Flupirtin verwendet.

2. Verfahren nach einem oder mehreren der vorangegangenen Ansprüche, dadurch gekennzeichnet, daß 10 bis 900 mg, vorzugsweise 10 bis 600 mg Flupirtin mit 0,3 bis 100 mg des Spasmolytikums zu einer Dosierungseinheit formuliert werden.

3. Verfahren zur Herstellung eines Arzneimittels, dadurch gekennzeichnet, daß man 1 Gewichtsteil Flupirtin und 0,05 bis 150 Gewichtsteile eines anticholingerisch wirkenden Spasmolytikums oder deren Salze mit physiologisch unbedenklichen Säuren zusammen mit üblichen Träger- und/oder Verdünnungs- beziehungsweise Hilfsstoffen zu einem Mittel verarbeitet, welches in der Dosierungseinheit 10 bis 900 mg Flupirtin und 0,3 bis 100 mg des Spasmolytikums enthält.

4. Verfahren zur Herstellung eines Arzneimittels, dadurch gekennzeichnet, daß man 1 Gewichtsteil Flupirtin und 0,05 bis 150 Gewichtsteile eines anticholinergisch wirkenden Spasmolytikums oder deren Salze mit physiologisch unbedenklichen Säuren zusammen mit üblichen Träger- und/oder Verdünnungs- beziehungsweise Hilfsstoffen bei Temperaturen zwischen 20 und 80 °C vermischt beziehungsweise homogenisiert, die so erhaltene Mischung zur Herstellung von Zubereitungen, die in der Dosierungseinheit 10 bis 900 mg Flupirtin und 0,3 bis 100 mg des Spasmolytikums enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

5. Verfahren zur Herstellung eines Mittels nach einem oder mehreren der vorangegangenen Ansprüche, dadurch gekennzeichnet, daß man 1 Gewichtsteil Flupirtin und 0,05 bis 150 Gewichtsteile eines anticholinergisch wirkenden Spasmolytikums oder deren Salze mit physiologisch unbedenklichen Säuren gegebenenfalls mit mindestens einem der Hilfsstoffe Stärke, Cellulose, Calciumhydrogenphosphat und modifizierte Stärke vermischt, mit einer wässrigen Gelatinelösung oder Stärkelösung oder einem wässrigen Vinylpyrrolidon-Vinylacetat-Copolymerisat granuliert und das erhaltene Granulat mit

Magnesiumstearat und hochdispersem Siliziumdioxid sowie gegebenenfalls auch Stärke und/oder Cellulose vermischt und zu Tabletten verpresst oder in Kapseln abfüllt.

6. Verfahren zur Herstellung eines Mittels nach einem oder mehreren der vorangegangenen Ansprüche, dadurch gekennzeichnet, daß man 1 Gewichtsteil Flupirtin und 0,05 bis 150 Gewichtsteile eines nicht-steroidalen Antiphlogistikums oder deren Salze mit physiologisch unbedenklichen Säuren, gegebenenfalls nach Zusatz von Sojalecithin, bei Temperaturen zwischen 33-37 °C in geschmolzenem Hartfett suspendiert und homogenisiert und anschließend die Mischung in Hohlzellen ausgießt.

7. Verwendung von Flupirtin und mindestens einem anticholinergisch wirkenden Spasmolytikum oder deren Salzen mit physiologisch unbedenklichen Säuren zur Herstellung eine analgetisch-spasmolytisch wirksamen Arzneimittels.

8. Verwendung von Flupirtin und mindestens einem anticholinergisch wirkenden Spasmolytikum oder deren Salzen mit physiologisch unbedenklichen Säuren zur Herstellung eines analgetisch-spasmolytisch wirksamen Arzneimittels, in dem 1 Gewichtsteil Flupirtin mit 0,05 bis 150 Gewichtsteilen des Spasmolytikums formuliert werden.

**Claims** (for the Contracting States : BE, CH, DE, FR, GB, IT, LI, LU, NL, SE)

1. Medicaments containing flupirtine and at least one anticholinergic spasmolytic, which has an analgesic side effect component, as active compound, it also being possible for these active compounds to be present in the form of their salts with physiologically acceptable acids, where to one part by weight of flupirtine in each case 0.05 to 150, preferably 0.1 to 100, parts by weight of spasmolytic are present.

2. Agents according to Claim 1, characterized in that the spasmolytic is a butylscopolaminium salt, fenpiverinium salt, trospium salt, pramiverine salt or ciclonium salt.

3. Agents according to one or more of the preceding claims, characterized in that the combination contains 10 to 900 mg of flupirtine and 0.3 to 100 mg of the spasmolytic.

4. Agents according to one or more of the preceding claims, characterized in that the combination contains 10 to 600 mg of flupirtine and 0.3 to 100 mg of the spasmolytic.

5. Agents according to one or more of the preceding claims, characterized in that the combination contains 30 to 600 mg of flupirtine and 3 to 60 mg of butylscopolamine (as the cation).

6. Agents according to one or more of the preceding claims, characterized in that the combination contains 30 to 400 mg of flupirtine and 3 to 60 mg of butylscopolamine (as the cation).

7. Agents according to one or more of the preceding claims, characterized in that the combination contains 10 to 900 mg of flupirtine and 5 to 60 mg of ciclonium base (as the cation).

**Claims** (for the Contracting State AT)

1. A process for the preparation of medicaments, characterized in that flupirtin and at least one anticholinergically active spasmolytic agent or salts thereof with physiologically acceptable acids, and optionally other conventional carriers and/or diluents or auxiliary agents are formulated into pharmaceutical formulations, characterized in that 1 part by weight of flupirtin is formulated with 0.05 to 150 parts by weight of the spasmolytic agent.

2. A process according to one or more of the preceding claims, characterized in that 10 to 900 mg, preferably 10 to 600 mg of flupirtin are formulated into a dosage unit with 0.3 to 100 mg of the spasmolytic agent.

3. A process for the preparation of a medicament, characterized in that 1 part by weight of flupirtin and 0.05 to 150 parts by weight of an anticholinergically active spasmolytic agent or salts thereof with physiologically acceptable acids together with conventional carriers and/or diluents or auxiliary agents are made up into a medicament which contains 10 to 900 mg of flupirtin and 0.3 to 100 mg of the spasmolytic agent per dosage unit.

4. A process for the preparation of a medicament, characterized in that 1 part by weight of flupirtin and 0.05 to 150 parts by weight of an anticholinergically active spasmolytic agent or salts thereof with physiologically acceptable acids are mixed or homogenized together with conventional carriers and/or diluents or auxiliary agents at temperatures between 20 and 80 °C, the mixture thereby obtained is poured into hollow moulds of appropriate size or filled into capsules of appropriate size or granulated and then optionally pressed into tablets with addition of other conventional auxiliary agents for the preparation of formulations which contain per dosage unit 10 to 900 mg of flupirtin and 0.3 to 100 mg of the spasmolytic agent.

5. A process for the preparation of a medicament according to one or more of the preceding claims, characterized in that 1 part by weight of flupirtin is mixed with 0.05 to 150 parts by weight of an anticholinergically active spasmolytic agent or salts thereof with physiologically acceptable acids and optionally with at least one of the auxiliary agents starch, cellulose, calcium hydrogen phosphate and modified starch, the product is granulated with an aqueous gelatine solution or starch solution or an aqueous vinyl pyrrolidone-vinyl acetate copolymerisate and the so-obtained granulate is mixed with

magnesium stearate and highly disperse silica as well as optionally also starch and/or cellulose and the product is pressed into tablets or filled into capsules.

6. A process for the preparation of a medicament according to one or more of the preceding claims, characterized in that 1 part by weight of flupirtin and 0.05 to 150 parts by weight of a non-steroidal antiinflammatory agent or salts thereof with physiologically acceptable acids and optionally after the addition of soybean lecithin are suspended and homogenized at temperatures between 33-37 °C in molten hard fat and the mixture is poured into hollow moulds.

7. The use of flupirtin and at least one anticholinergically active spasmolytic agent or salts thereof with physiologically acceptable acids for the preparation of an analgesically-spasmolytically active medicament.

8. The use of flupirtin and at least one anticholinergically active spasmolytic agent or salts thereof with physiologically acceptable acids for the preparation of an analgesically-spasmolytically acting medicament in which 1 part by weight of flupirtin is formulated with 0.05 to 150 parts by weight of the spasmolytic agent.

**Revendications** (pour les Etats contractants : BE, CH, DE, FR, GB, IT, LI, LU, NL, SE)

1. Médicament contenant, en tant que substance active, de la flupirtine et au moins un spasmolytique à action anticholinergique qui présente une composante d'effet secondaire analgésique, ces substances actives pouvant également se présenter sous forme de leurs sels avec des acides physiologiquement inoffensifs et le spasmolytique étant présent dans des proportions de 0,05 à 150, de préférence de 0,1 à 100 parties en poids pour 1 partie en poids de flupirtine.

2. Médicament selon la revendication 1, caractérisé en ce que le spasmolytique est un sel de butylscopolaminium, un sel de fenpiverinium, un sel de trospium, un sel de pramivérine ou un sel de ciclonium.

3. Médicament selon la revendication 1 ou 2, caractérisé en ce que la combinaison contient de 10 à 900 mg de flupirtine et de 0,3 à 100 mg du spasmolytique.

4. Médicament selon l'une quelconque des revendications 1 à 3, caractérisé en ce que la combinaison contient de 10 à 600 mg de flupirtine et de 0,3 à 100 mg du spasmolytique.

5. Médicament selon l'une quelconque des revendications 1 à 4, caractérisé en ce que la combinaison contient de 30 à 600 mg de flupirtine et de 3 à 60 mg de butylscopolamine (en tant que cation).

6. Médicament selon l'une quelconque des revendications 1 à 5, caractérisé en ce que la combinaison contient de 30 à 400 mg de flupirtine et de 3 à 60 mg de butylscopolamine (en tant que cation).

7. Médicament selon l'une quelconque des revendications 1 à 6, caractérisé en ce que la combinaison contient de 10 à 900 mg de flupirtine et de 5 à 60 mg de ciclonium-base (en tant que cation).

**Revendications** (pour l'Etat contractant AT)

1. Procédé de préparation de médicaments, caractérisé en ce qu'on formule de la flupirtine et au moins un spasmolytique à action anticholinergique ou des sels de celui-ci avec des acides physiologiquement inoffensifs, le cas échéant avec d'autres excipients et/ou diluants ou adjuvants usuels, sous forme de préparations pharmaceutiques, en utilisant de 0,05 à 150 parties en poids de spasmolytique pour 1 partie en poids de flupirtine.

2. Procédé selon la revendication 1, caractérisé en ce que 10 à 900 mg, de préférence 10 à 600 mg de flupirtine sont formulés avec 0,3 à 100 mg du spasmolytique pour une unité posologique.

3. Procédé de préparation d'un médicament, caractérisé en ce qu'on met sous forme de médicament 1 partie en poids de flupirtine et 0,05 à 150 parties en poids d'un spasmolytique à action anticholinergique ou des sels de celui-ci avec des acides physiologiquement inoffensifs, en combinaison avec des excipients et/ou diluants ou adjuvants usuels, de 10 à 900 mg de flupirtine et de 0,3 à 100 mg du spasmolytique étant contenus dans l'unité posologique.

4. Procédé de préparation d'un médicament, caractérisé en ce qu'on mélange ou homogénéise, à des températures comprises entre 20 et 80 °C, 1 partie en poids de flupirtine et 0,05 à 150 parties en poids d'un spasmolytique à action anticholinergique ou des sels de celui-ci avec des acides physiologiquement inoffensifs, en combinaison avec des excipients et/ou diluants ou adjuvants usuels, on coule dans des alvéoles creux de taille appropriée, on charge dans des capsules de taille appropriée ou on granule le mélange ainsi obtenu pour la confection de préparations qui contiennent 10 à 900 mg de flupirtine et 0,3 à 100 mg du spasmolytique par unité posologique, puis on le presse éventuellement en comprimés avec addition d'autres adjuvants usuels.

5. Procédé de préparation d'un médicament selon l'une quelconque des revendications 1 à 4, caractérisé en ce qu'on mélange 1 partie en poids de flupirtine et 0,05 à 150 parties en poids d'un spasmolytique à action anticholinergique ou des sels de celui-ci avec des acides physiologiquement

inoffensifs, éventuellement avec au moins l'un des adjuvants amidon, cellulose, hydrogénophosphate de calcium et amidon modifié, on granule le mélange avec une solution aqueuse de gélatine, une solution d'amidon ou un copolymère vinylpyrrolidone/acétate de vinyle aqueux, on mélange le granulé obtenu avec du stéarate de magnésium, du bioxyde de silicium fortement dispersé et éventuellement de l'amidon et/ou de la cellulose et on le presse en comprimés ou on le charge dans des capsules.

6. Procédé de préparation d'un médicament selon l'une quelconque des revendications 1 à 5, caractérisé en ce qu'on met en suspension et on homogénéise dans de la graisse dure fondue, à une température comprise entre 33 et 37 °C, 1 partie en poids de flupirtine et 0,05 à 150 parties en poids d'un antiphlogistique non stéroïde ou des sels de celui-ci avec des acides physiologiquement inoffensifs, le cas échéant après addition de lécithine de soja, puis on coule le mélange dans des alvéoles creux.

7. Utilisation de flupirtine et d'au moins un spasmolytique à action anticholénergique ou de sels de celui-ci avec des acides physiologiquement inoffensifs pour la préparation d'un médicament à action analgésique-spasmolytique.

8. Utilisation de flupirtine et d'au moins un spasmolytique à action anticholénergique ou de sels de celui-ci avec des acides physiologiquement inoffensifs pour la préparation d'un médicament à action analgésique-spasmolytique dans lequel 1 partie en poids de flupirtine est formulée avec 0,05 à 150 parties en poids du spasmolytique.